# EUROPÄISCHE PATENTSCHRIFT

(11) **EP 0 699 447 B1**
(45) Veröffentlichungstag und Bekanntmachung des Hinweises auf die Patenterteilung: **14.11.2001**
(21) Anmeldenummer: 95108362.5
(22) Anmeldetag: 01.06.1995
(51) Int. Cl.: A61M 1/10

(54) **Zentrifugal-Blutpumpe**
Centrifugal bloodpump
Pompe à sang centrifuge

(30) Priorität: 31.08.1994 DE 4430853
(43) Veröffentlichungstag der Anmeldung: 06.03.1996
(73) Patentinhaber: Jostra AG, 72145 Hirrlingen (DE)
(72) Erfinder: Mendler, Nikolaus, Prof. Dr. med., D-82335 Berg (DE)
(74) Vertreter: Möbus, Daniela, Dr.-Ing.

(56) Entgegenhaltungen:
- WO-A-92/03181
- AT-B- 393 456
- DE-A- 3 133 177
- GB-A- 1 383 811
- US-A- 4 984 972
- US-A- 5 174 726

## Beschreibung

Die Erfindung betrifft eine Zentrifugal-Blutpumpe, insbesondere für Herz-Ersatzeinrichtungen, mit einem innerhalb eines his auf mindestens eine Bluteinlass- und mindestens eine Blutauslassöffnung flüssigkeits- und gasdicht geschlossenen Gehäuses drehbar angeordneten Pumpenrotor, der von einem außerhalb angeordneten Antriebsmotor mittels magnetischer Kopplung antreibbar ist.

Zentrifugal-Blutpumpen gewinnen gegenüber so genannten Rollerpumpen und auch gegenüber Ventrikelpumpen für die Blutförderung immer mehr an Bedeutung, da mit ihnen bei der Blutförderung auftretende Nachteile der vorstehend genannten Pumpen vermieden werden können. Diese Nachteile bestehen bei der leicht zu bedienenden und kostengünstigen Rollerpumpe darin, dass es sich hier um eine reine Verdrängerpumpe handelt, die gegenüber dem gepumpten Medium unsensibel ist, sodass beispielsweise beim Leerlaufen eines vorgeschalteten Blutreservoirs Luft mit tödlicher Folge in einen Patienten gepumpt werden kann. Außerdem kann sich bei einer Blockierung einer Blutleitung ein beliebig hoher Druck in dem System aufbauen. Durch eine ständige Walkarbeit der Roller auf einem blutführenden Schlauch ist mechanische Hämolyse unvermeidlich und kann im Innern des Schlauchs eine Emboliegefahr bedeutender Abrieb entstehen.

Bei der Ventrikelpumpe ist neben der ebenfalls möglichen Förderung von Luft der Hauptnachteil darin zu sehen, dass sie eine aufwendige Konstruktion mit Membranen und Ventilen darstellt, entsprechend teuer ist und sich daher nicht gut zu einer aus Sicherheitsgründen erforderlichen Einwegeinrichtung eignet.

Demgegenüber ist bei den Zentrifugal-Blutpumpen der Vorteil vorhanden, dass bei einem massiven Lufteinbruch die Pumpwirkung sofort unterbrochen wird. Auch muss nicht mit einem mechanischen Abrieb von Kunststoffpartikeln und deren Verschleppung in den Blutstrom gerechnet werden. Die Blutschädigung durch Rotationspumpen ist merklich geringer als durch Rollerpumpen.

Die beispielsweise aus der WO 92/03181 und US-A-5 017 103 bekannten Zentrifugalpumpen für die Förderung von Blut in der extrakorporalen Zirkulation haben aber immer noch den Nachteil, dass sie konstruktiv aufwendig sind, auch in ihrem als Einmal-Artikel auszuführenden Pumpenteil. Für den Rotor sind außerhalb des blutführenden Bereiches befindliche Lager vorgesehen, die über Wellendichtungen gegen den blutführenden Bereich abgedichtet sein müssen. Die Dichtungen begrenzen die Standzeit der Blutpumpen. Weitere Nachteile sind eine mangelhafte Abfuhr von Reibungswärme, die bei einer längeren Verweilzeit des Bluts innerhalb der Pumpe zu einer lokalen Überhitzung von Blut mit der Gefahr von Denaturierung von Blutei-weiß und einer Zellzerstörung führen kann.

Aus der US-A-4 984 972 ist eine dichtungsfreie Zentrifugal-Blutpumpe bekannt, bei der die Magnete des Antriebs jedoch diagonal zur Drehachse des Pumpenrotors angeordnet sind, wodurch eine starke axiale Komponente der Magnetkraft entsteht, die vom Rotorlager aufgefangen werden muss. Dadurch kann es zu einer lokalen Erwärmung im Lagerbereich sowie zu einem vorzeitigen Verschleiß des Lagers kommen.

Die AT 393 456 B beschreibt eine Blutpumpe, bei der der Pumpenrotor mittels eines magnetischen Wechselfeldes, das mittels Spulen in einem Stator erzeugt wird, angetrieben wird. Dabei sind jedoch Kugellager und Wellendichtungen zur Führung erforderlich. Für eine Anwendung in der extrakorporalen Zirkulation mit einer Herz-Lungen-Maschine sind diese Pumpen ungeeignet, da sie bei Stromausfall nicht auf Handbetrieb umstellbar sind.

Es sind auch bereits dichtungsfreie Zentrifugal-Blutpumpen und selbst solche mit lagerlosen Rotoren mit ausschließlich magnetischer Stabilisierung vorgeschlagen worden. Diese Pumpen haben aber immer noch den Nachteil eines großen Konstruktionsaufwandes, und sie erfordern aus Stabilisierungsgründen eine aufwendige Steuerung mit elektromagnetischer Rückkoppelung und damit einen unerwünscht großen Pumpenrotor, was wiederum zu einem großen Blutkammervolumen dieser Pumpen mit entsprechend großer Reibkontaktfläche für das Blut und einer relativ langen Verweilzeit des Blutes im Pumpenbereich führt.

Der Erfindung liegt die Aufgabe zugrunde, eine ZentrifugalBlutpumpe der eingangs genannten Art so auszubilden, dass sie unter Vermeidung vorstehend genannter Nachteile eine hohe Standzeit aufweist.

Die gestellte Aufgabe wird mit einer Zentrifugal-Blutpumpe mit den im Hauptanspruch aufgeführten Merkmalen gelöst.

Die angestrebte Bauweise des Pumpenteils wird vor allem durch die radial gerichtete magnetische Mitnahmekopplung des Pumpenrotors mit einem äußeren Antriebsteil und durch die Vermeidung aufwendiger und teurer Stabilisierungsmagnete im Bereich des Pumpenrotors erzielt. Außerdem ist eine zusätzliche, im Blutdurchlaufbereich dichtungsfrei ausgebildete Gleitlagerung des Rotors vorgesehen. Dabei lassen sich die permanent magnetisierten oder auch nicht permanent magnetisierten ferromagnetischen Bereiche des Pumpenrotors auf eine sehr geringe Höhe begrenzen, sodass das Verhältnis des Radius des Mitnehmerteils des Pumpenrotors zur Höhe der ferromagnetischen Bereiche mindestens 10:1 betragen kann. Entsprechend lässt sich auch der außerhalb des Pumpengehäuses angeordnete und mit einem Antriebsmotor verbundene Drehkupplungsteil mit geringer axialer Höhe ausbilden. Zweckmäßig ist der Mitnehmerteil des Pumpenrotors scheibenförmig ausgebildet und auf mindestens einer der beiden Seiten dieses scheibenförmigen Teils mit Mitnahmeelementen zur Förderung des Bluts versehen.

Versuche mit einem ersten Prototyp haben bereits gezeigt, dass sich eine erfindungsgemäß ausgebildete Zentrifugal-Blutpumpe mit ihrem relativ geringen Kostenaufwand ihres Einweg-Pumpenteils betriebssicher und mit einem relativ kleinen Pumpenkammervolumen ausbilden lässt, sodass die Kontaktfläche, die das Blut mit dem Pumpenteil hat, entsprechend gering ist und die Verweilzeit des Bluts in der Pumpeinrichtung wesentlich kürzer ist, als dies bei bisher bekannten Zentrifugal-Blutpumpen der Fall ist. Der Wirkungsgrad der erfindungsgemäß ausgebildeten Pumpe ist dementsprechend hoch. An einem Schneiden- oder Spitzenlager kann bei dem raschen Blutdurchsatz durch die Pumpe keine Übererwärmung und keine merkliche mechanische Hämolyse erfolgen. Die Kombination einer guten magnetischen Stabilisierung in Axialrichtung mit einer zusätzlichen mechanischen Stabilisierung des Pumpenrotors in Radialrichtung stellt keinen nachteiligen Kompromiß dar, sondern bringt bei dem bereits schon erwähnten kostengünstigen geringen Konstruktionsaufwand eine wesentliche Erhöhung der Betriebssicherheit gegenüber einer aufwendigen, rein magnetischen Stabilisierung des Rotors einer solchen Pumpe. Bei Stromausfall ist die Stabilisierung des Rotors nicht gestört, sodass die Pumpe im Handbetrieb weiter betätigt werden kann.

Nachfolgend wird ein Ausführungsbeispiel einer erfindungsgemäß ausgebildeten Zentrifugal-Blutpumpe anhand der beiliegenden Zeichnung näher erläutert.

Im Einzelnen zeigen:
- Fig. 1: einen zentralen Längsschnitt durch die Zentrifugal-Blutpumpe;
- Fig. 2: einen Querschnitt durch die Blutpumpe entlang der Linie II-II in Fig. 1;
- Fig. 3: einen Querschnitt durch die Blutpumpe entlang der Linie III-III in Fig. 1;
- Fig. 4: eine perspektivische Einzeldarstellung des magnetischen Mitnehmer- und Stabilisierungssystems für den Pumpenrotor.

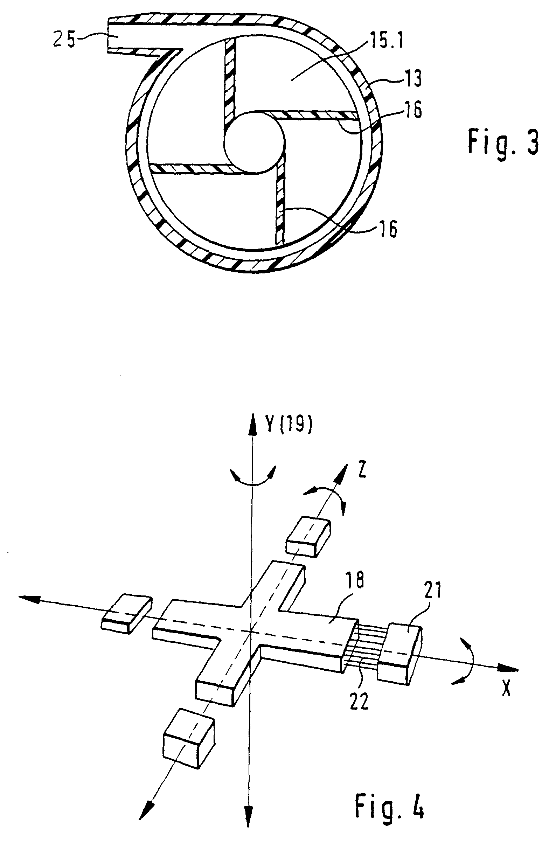

Aus der stark schematisierten Schnittfigur 1 sind ein elektrischer Antriebsmotor 10, seine Abtriebswelle 11, eine auf der Abtriebswelle 11 konzentrisch befestigte Scheibe 12 und ein in einem nicht dargestellten Halter auswechselbar und stationär angeordnetes Pumpengehäuse 13 ersichtlich. Das Pumpengehäuse 13 ist bis auf eine zentrale Pumpeneinlaßöffnung 14 und eine aus Fig. 3 ersichtliche tangentiale Pumpenauslauföffnung 25 dicht verschlossen und bildet ein in einen nicht dargestellten extrakorporalen Abschnitt eines Blutkreislaufs einsetzbares Betriebsteil, das nur für eine einmalige Benutzung vorgesehen ist.

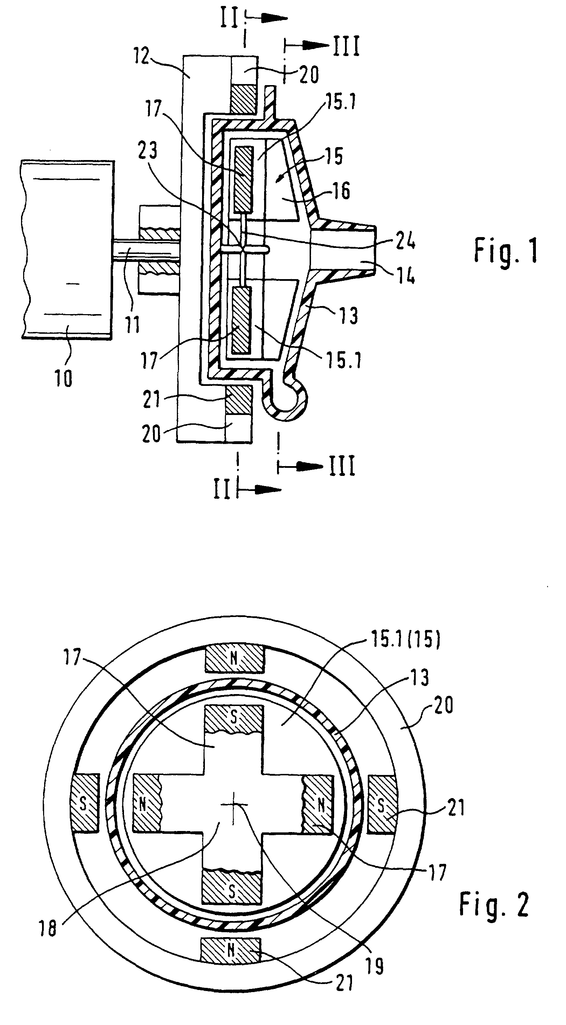

Im Innern des dichten Pumpengehäuses 13 ist ein Pumpenrotor 15 angeordnet, der aus einem scheibenförmigen Mitnehmerteil 15.1 besteht, an dessen einer Seite Pumpelemente 16 passender und auch bekanntere Art angeformt sind, insbesondere gekrümmte Flügel eines offenen Flügelrades. In dem scheibenförmigen Mitnehmerteil 15.1 sind in gleichmäßiger Verteilung über seinen Umfang ferromagnetische Bereiche 17 ausgebildet, die beim dargestellten Ausführungsbeispiel permanentmagnetisiert sind. Wie das Schnittbild der Fig. 2 zeigt, sind bei dem dargestellten Ausführungsbeispiel die ferromagnetischen Bereiche 17 die Arme eines gleicharmigen Kreuzes 18, und dieses ferromagnetische Kreuz 18 ist so permanentmagnetisiert, daß seine vier Arme über den Rotorumfang gesehen aufeinanderfolgende Nordpole N und Südpole S bilden. Die Rotationsachse 19 des Pumpenrotors 15 verläuft koaxial zur Abtriebswelle 11 des mit einer nicht dargestellten elektrischen Steuereinrichtung gekoppelten Antriebsmotors 10.

Auf der mit der Abtriebswelle 11 des Motors 10 verbundenen Scheibe 12 ist ein das Pumpengehäuse 13 auf der Höhe des Mitnehmerteiles 15.1 des Pumpenrotors 15 umfangender Ring 20 angeordnet, der gemäß Fig. 2 bei dem dargestellten Ausführungsbeispiel auf seiner Innenseite mit vier Permanentmagneten 21 besetzt ist, die über den Umfang des Pumpenrotors 15 und seines Mitnehmerteiles 15.1 gesehen abwechselnd Nordpole N und Südpole S bilden. Die Permanentmagnete 21 laufen mit konstantem Abstand zusammen mit dem eine magnetische Rückschlußbrücke bildenden Ring 20 um das stationäre Pumpengehäuse 13 und nehmen dabei den Pumpenrotor 15 durch magnetische Einwirkung auf das ferromagnetische Kreuz 18 seines Mitnehmerteils 15.1 mit. Gleichzeitig wird dabei auch eine Stabilisierung des Pumpenrotors in vier von sechs räumlichen Freiheitsgraden bewirkt.

Fig. 4 zeigt das Prinzip der eine Mitnahme des Pumpenrotors 15 um die Drehachse 19 bewirkenden magnetische Koppelung zwischen den umlaufenden Permanentmagneten 21 und dem ferromagnetischen Kreuz 18 des Pumpenrotors. Fig. 4 zeigt auch, daß das ferromagnctische Kreuz 18 und die umlaufenden Permantentmagneten 21 jeweils als dünne Platte oder Plättchen ausgebildet sind, wobei die umlaufenden Permanentmagnetplättchen 21 jeweils eine Magnetfeldbrücke 22 zu den Armen des ferromagnetischen Kreuzes 18 bilden, die so flach ist, daß durch sie keine merklichen Kippmomente auf den zugeordneten Arm des ferromagnetischen Kreuzes 18 ausgeübt werden, vielmehr durch die gleichmäßige Verteilung der Magnetfeldbrücken 22 über den Umfang des Pumpcnrotors und seines ferromagnetischen Kreuzes 18 eine Stabilisierung des Pumpenrotors erfolgt. Sowohl bei dem die Permanentmagnete 21 innenseitig tragenden Drehkupplungsteil als auch bei dem Pumpenrotor 15 beträgt das Maßverhältnis des Radius dieser Teile zu der Höhe der Permanentmagnete 21 oder des ferromagnetischen Kreuzes 18 mindestens 10:1.

Bei dem dargestellten Ausführungsbeispiel einer Zentrifugal-Blutpumpe gemäß der Erfindung ist der Rotor 15 trotz seiner magnetischen Stabilisierung in vier von sechs Freiheitsgraden zusätzlich innerhalb des Gehäuses 13 an der in Fig. 1 angedeuteten zentralen Stelle 23 mittels einer Scheibe 24 mit einer einen schneidenförmigen Rand aufweisenden Lageröffnung quasi spitzengelagert und dadurch gegen translatorische Bewegungen in der x-z-Ebene (Fig. 4) mechanisch stabilisiert.

## Patentansprüche

1. Zentrifugal-Blutpumpe, insbesondere für Herz-Ersatzeinrichtungen, mit einem innerhalb eines bis auf mindestens eine Bluteinlass- und mindestens eine Blutauslassöffnung (14, 25) flüssigkeits- und gasdicht geschlossenen Gehäuses (13) drehbar angeordneten Pumpenrotor (15), der von einem außerhalb angeordneten Antriebsmotor (10) mittels magnetischer Kopplung antreibbar ist, **dadurch gekennzeichnet, dass** der Pumpenrotor (15) von seiner Peripherie her beeinflussbare, über den Rotorumfang gleichmäßig verteilte ferromagnetische Bereiche (17, 18) aufweist, auf welche radial senkrecht zur Drehachse (19) des Pumpenrotors (15) ausgerichtete, außerhalb des Gehäuses in einem mit dem Antriebsmotor (10) verbundenen Drehkupplungsteil (12/20) ebenfalls über dessen Umfang gleichmäßig verteilt angeordnete Permanentmagnete (21) den Pumpenrotor mitnehmend und stabilisierend einwirken, wobei die Höhe der Permanentmagnete (21) des Drehkupplungsteils (12/20) in Axialrichtung des Pumpenrotors (15) mindestens annähernd gleich der Höhe der ferromagnetischen Bereiche (17, 18) des Pumpenrotors (15) ist; dass zur zusätzlichen Stabilisierung gegen Translation in Radialrichtung der Pumpenrotor (15) innerhalb des Pumpengehäuses (13) im Blutdurchlaufbereich dichtungsfrei mechanisch gelagert ist und das mechanische Lager des Pumpenrotors aus einem eine Schneide oder Spitzen aufweisenden Lagerteil (24) besteht.

2. Zentrifugal-Blutpumpe nach Anspruch 1, **dadurch gekennzeichnet, daß** der Drehkupplungsteil (12/20) einen die Permanentmagnete (21) auf seiner Innenumfangsseite tragenden Ring (20) aufweist, der gleichzeitig eine magnetische Rückschlussbrücke zwischen den einzelnen Permanentmagneten bildet.

3. Zentrifugal-Blutpumpe nach Anspruch 1 oder 2, **dadurch gekennzeichnet, dass** sowohl bei dem Drehkupplungsteil (20) an seinem Innenumfang als auch bei dem Pumpenrotor (15.1) an seinem Außenumfang das Verhältnis des Radius dieser Teile zu der Höhe der Permanentmagnete (21) oder der ferromagnetischen Bereiche (17, 18) mindestens 10:1 beträgt.

4. Zentrifugal-Blutpumpe nach einem der Ansprüche 1 bis 3, **dadurch gekennzeichnet, dass** die ferromagnetischen Bereiche (17) des Pumpenrotors (15) permanent magnetisiert sind.

5. Zentrifugal-Blutpumpe nach einem der Ansprüche 1 bis 4, **dadurch gekennzeichnet, dass** die Anzahl der Permanentmagnete (21) des Drehkupplungsteils (12/20) gleich der Anzahl der ferromagnetischen Bereiche (17) des Pumpenrotors ist.

6. Zentrifugal-Blutpumpe nach einem der Ansprüche 1 bis 5, **dadurch gekennzeichnet, dass** die ferromagnetischen Bereiche (17, 18) des Pumpenrotors (15) in einem scheibenförmigen Mitnehmerteil (15.1) des Rotorkörpers ausgebildet sind, an welchen sich mindestens axial einseitig ein mit Pumpelementen (16) versehener Rotorteil anschließt.

## Claims

1. A centrifugal blood pump, particularly for heart substitute devices, having a pump rotor (15) which is rotatably disposed inside a housing (13) which is closed in a liquid- and gas-tight manner except for at least one blood inlet opening and at least one blood outlet opening (14, 25), which rotor can be driven by an externally situated drive motor (10) by means of magnetic coupling, **characterised in that** the pump rotor (15) comprises ferromagnetic regions (17, 18), which can be acted upon from its periphery and which are uniformly distributed over the rotor periphery, and on which permanent magnets (21) act with a driving and stabilising effect, which permanent magnets are disposed outside the housing in a rotary coupling part (12/20) which is attached to the drive motor (10) and are likewise uniformly distributed over the periphery of the rotary coupling part, and are aligned radially perpendicularly to the axis of rotation (19) of the pump rotor (15), wherein the height of the permanent magnets (21) of the rotary coupling part (12/20) in the axial direction of the pump rotor (15) is at least approximately equal to the height of the ferromagnetic regions (17, 18) of the pump rotor (15), that for additional stabilisation from translational motion in a radial direction, the pump rotor (15) is mechanically mounted without a seal inside the pump housing (13) in the region for the passage of blood, and the mechanical bearing of the pump rotor consists of a bearing part (24) comprising a knife edge or points.

2. A centrifugal blood pump according to claim 1, **characterised in that** the rotary coupling part (12/20) comprises a ring (20) which bears the permanent magnets (21) on its inner peripheral face and which at the same time forms a magnetic back-connection bridge between the individual permanent magnets.

3. A centrifugal blood pump according to claims 1 or 2, **characterised in that** both at the inner periphery of the rotary coupling part (20) and at the outer periphery of the pump rotor (15.1) the ratio of the radii of these parts to the height of the permanent magnets (21) or to the ferromagnetic regions (17, 18) is at least 10:1.

4. A centrifugal blood pump according to any one of claims 1 to 3, **characterised in that** the ferromagnetic regions (17) of the pump rotor (15) are permanently magnetised.

5. A centrifugal blood pump according to any one of claims 1 to 4, **characterised in that** the number of permanent magnets (21) of the rotary coupling part (12/20) is equal to the number of ferromagnetic regions (17) of the pump rotor.

6. A centrifugal blood pump according to any one of claims 1 to 5, **characterised in that** the ferromagnetic regions (17, 18) of the pump rotor (15) are formed in a disc-shaped driver part (15.1) of the rotor body, which is adjoined at least axially on one side by a rotor part which is provided with pump elements (16).

## Revendications

1. Pompe à sang centrifuge, en particulier pour dispositifs de remplacement du coeur, avec un rotor de pompe (15) qui est monté tournant à l'intérieur d'un boîtier (13) fermé de manière étanche aux liquides et aux gaz à l'exception d'au moins un orifice d'entrée de sang et d'au moins un orifice de sortie de sang (14, 25) et qui, au moyen d'un accouplement magnétique, peut être entraîné par un moteur d'entraînement (10) disposé à l'extérieur, **caractérisée en ce que** le rotor de pompe (15) comporte des zones ferromagnétiques (17, 18) qui sont uniformément réparties sur le pourtour du rotor et sont influençables à partir de sa périphérie et sur lesquelles des aimants permanents (21) orientés radialement perpendiculairement à l'axe de rotation (19) du rotor de pompe (15) et eux aussi uniformément répartis à l'extérieur du boîtier dans une pièce d'accouplement en rotation (12/20) reliée au moteur d'entraînement (10) et sur le pourtour de celle-ci agissent en entraînant et en stabilisant le rotor de pompe, la hauteur des aimants permanents (21) de la pièce d'accouplement en rotation (12/20) dans la direction axiale du rotor de pompe (15) étant au moins approximativement égale à la hauteur des zones ferromagnétiques (17, 18) du rotor de pompe (15), **en ce que**, pour la stabilisation supplémentaire contre la translation dans la direction radiale, le rotor de pompe (15) est supporté mécaniquement sans joint à l'intérieur du boîtier de pompe (13) dans la zone de passage du sang, et **en ce que** le support mécanique du rotor de pompe se compose d'une pièce de support (24) comportant une lame ou des pointes.

2. Pompe à sang centrifuge selon la revendication 1, **caractérisée en ce que** la pièce d'accouplement en rotation (12/20) comporte un anneau (20) qui porte les aimants permanents (21) sur son côté périphérique intérieur et qui forme en même temps un pont de couplage magnétique entre les aimants permanents individuels.

3. Pompe à sang centrifuge selon la revendication 1 ou 2, **caractérisée en ce que**, aussi bien dans la pièce d'accouplement en rotation (20) sur son pourtour intérieur que dans le rotor de pompe (15.1) sur son pourtour extérieur, le rapport du rayon de ces pièces à la hauteur des aimants permanents (21) ou des zones ferromagnétiques (17, 18) est d'au moins 10:1.

4. Pompe à sang centrifuge selon l'une des revendications 1 à 3, **caractérisée en ce que** les zones ferromagnétiques (17) du rotor de pompe (15) sont magnétisées de manière permanente.

5. Pompe à sang centrifuge selon l'une des revendications 1 à 4, **caractérisée en ce que** le nombre des aimants permanents (21) de la pièce d'accouplement en rotation (12/20) est égal au nombre de zones ferromagnétiques (17) du rotor de pompe.

6. Pompe à sang centrifuge selon l'une des revendications 1 à 5, **caractérisée en ce que** les zones ferromagnétiques (17, 18) du rotor de pompe (15) sont réalisées dans une pièce d'entraînement en forme de disque (15.1) du corps de rotor, à laquelle une pièce de rotor munie d'éléments de pompe (16) se raccorde au moins axialement d'un côté.
